(19) Europäisches Patentamt
European Patent Office
Office européen des brevets

(11) Numéro de publication: **0 001 190**
**B1**

(12) # FASCICULE DE BREVET EUROPEEN

(45) Date de publication du fascicule de brevet: **25.02.81**

(51) Int. Cl.³: **C 08 L 5/14,** A 61 L 9/01, A 61 L 9/04

(21) Numéro de dépôt: **78400071.3**

(22) Date de dépôt: **02.08.78**

(54) **Utilisation de gels de carboxyméthylgalactomannane comme supports pour produits de traitement de l'air.**

(30) Priorité: **31.08.77 FR 7726435**

(43) Date de publication de la demande: **21.03.79 Bulletin 79/6**

(45) Mention de la délivrance du brevet: **25.02.81 Bulletin 81/8**

(84) Etats contractants désignés: **CH DE GB NL**

(56) Documents cités:
**US-A-2 599 771**
**US-A-3 301 723**
**US-A-3 615 794**
**US-A-3 677 961**
**US-A-3 723 409**
**US-A-3 888 312**

(73) Titulaire: **C E C A S.A., 11, Avenue Morane Saulnier, F-78140 - Velizy Villacoublay (FR)**

(72) Inventeur: **Brigand, Gérard, Cité Auby, F-50500 Carentan (FR)**
Inventeur: **Kragen, Horst, Cité Auby, F-50500 Carentan (FR)**

(74) Mandataire: **Petit, Alain et al, OFFICE JOSSE & PETIT 126 Boulevard Haussmann, F-75008 Paris (FR)**

EP 0 001 190 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

BUNDESDRUCKEREI BERLIN

# Utilisation de gels de carboxyméthylgalactomannane comme supports pour produits de traitement de l'air

La présente invention concerne l'utilisation comme gel support de produit de traitement de l'air d'une composition contenant comme agent gélifiant principal un galactomannane carboxyméthylé réticulé par un cation métallique trivalent.

Les gels pour traitement de l'air constituent un support par exemple pour parfums, désodorisants, insecticides, bactéricides, désinfectants qui, placé dans un local, permet de traiter l'atmosphère par exemple en supprimant les mauvaises odeurs ou les insectes; le support doit être tel qu'il laisse dégager progressivement les parfums, désodorisants ou autres afin que son efficacité soit prolongée; il est apparu que les gels de polysaccharides permettaient d'obtenir cette efficacité prolongée et on a proposé d'utiliser des dérivés des algues tels que les alginates et les carraghénanes.

Récemment, on a proposé d'utiliser comme agent gélifiant, dans la préparation de gels pour le traitement de l'air, le produit de réaction de la carboxyméthyl cellulose et d'un sel métallique. Le brevet anglais 1 448 318 décrit l'utilisation comme sel métallique d'un sel de métal alcalin ou d'un sel de métal trivalent tel que l'aluminium. On trouve aussi décrite l'utilisation de carboxyméthyl cellulose réticulée par un sel de métal trivalent tel que l'aluminium et le chrome dans les demandes de brevets français 2 302 750 et 2 326 937.

La réticulation de la carboxyméthylcellulose apparaît donner des gels intéressants du fait que leur formation ne nécessite pas de chauffage et qu'ils présentent une bonne aptitude à ne pas présenter le phénomène de synérèse. Toutefois, une cartaine élasticité du gel et une assez forte rétention peuvent être un obstacle à l'utilisation des modes de présentation habituels.

Par ailleurs, le brevet américain 2 599 771 décrit la préparation de gels aqueux à partir de sels alcalins de carboxyméthylgalactomannanes et de sels de métaux polyvalents tels que le fer et l'aluminium; aucune utilisation particulière n'est suggérée.

Il a été trouvé, et ceci est l'objet de l'invention, qu'on obtenait des gels convenant pour traitement de l'air, dont la formation s'effectue à la température ambiante, ne présentant pas de synérèse et ayant les qualités voulues de cohésion et de rétention pour assurer la diffusion de parfums dans les meilleures conditions en utilisant, dans certaines conditions, comme agent gélifiant un carboxyméthylgalactomannane réticulé par un sel de chrome.

Les galactomannanes se trouvent à l'état naturel et sont notamment connus sous les noms de gomme de guar (Cyamopsis tetragonolobus), gomme de caroube (Ceratonia siliqua), gomme tara (Caesalpinia spinosa), gomme d'Espina corona (Gleditsia amorphoïdes).

On rappellera que:

Les galactomannanes sont formés d'une chaîne principale de motifs D-mannose liés $\beta$ (1 → 4) avec des motifs D-galactose branchés sur cette chaîne par des liaisons $\alpha$ (1 → 6).

En remplaçant des hydrogènes des groupements hydroxyle d'un galactomannane par des groupements carboxyméthylés, on obtient un dérivé, le galactomannane carboxyméthylé ou carboxyméthyl galactomannane.

Le galactomannane carboxyméthylé peut être sous forme de sels de sodium ou de potassium et est produit notamment par réaction entre un galactomannane en mulieu alcalin et le monochloraceétate de sodium ou de potassium dans des conditions bien défnies et suivant des procédés connus.

La réaction de carboxyméthylation est la suivante:

$$R - OH + ClCH_2COONa + NaOH \rightarrow R - OCH_2COONa + NaCl + H_2O$$

Il a été trouvé que l'on pouvait produire un gel stable en utilisant une composition contenant de 0,5% à 10% en poids de galactomannane carboxyméthylé, une source de chrome trivalent, en quantité telle que le rapport pondéral métal trivalent contenu dans le sel sur la quantité de galactomannane carboxyméthylé soit compris entre 0,005 et 0,1 et de l'eau.

On définit un degré de substitution D.S comme étant le nombre moyen d'hydroxyles substitués par du carboxyméthyle dans un motif monomère de galactose ou de mannose.

Comme il y a en moyenne 3 hydroxyles libres par motif monomère, le D.S peut théoriquement atteindre 3, mais dans la pratique il est très difficile de dépasser 2,5.

Néanmoins, afin d'obtenir de bonnes caractéristiques de gel sans synérèse, l'optimum se situe entre 0,2 et 0,5.

Il s'est révélé selon l'une des caractéristiques de l'invention qu'au-dessous de 0,2 on obtient des gels souples mais peu rigides, même avec des doses importantes en cations trivalents alors qu'au-dessus de 0,5 on risque d'obtenir des gels rigides et cassants avec formation possible de synérèse.

Il est possible de faire varier la viscosité d'un galactomannane carboxyméthylé afin de l'adapter aux techniques de fabrication des gels (notamment pour le traitement de l'air) mais il est préférable d'utiliser un galactomannane carboxyméthylé de bas poids moléculaire (faiblement visqueux) plutôt qu'un galactomannane carboxyméthylé de haut poids moléculaire (très visqueux qui gélifie à une concentration plus faible, ceci afin d'améliorer les caractéristiques des gels: cohésion plus grande et absence de synérèse.

Tous les sels de métaux trivalents peuvent conduire à la réticulation. Cependant, il est apparu préférable pour obtenir une meilleure cohésion du gel d'utiliser plus particulièrement les sels de chrome, seuls ou en mélange. Ces sels peuvent être notamment les sulfates, les sulfates basiques, les chlorures et les acétates de chrome ainsi que les aluns de chrome.

A noter que certains anions sont particulièrement utiles car ils limitent la libération des cations et retardent ainsi la formation du gel.

Pour la fabrication du gel, on prépare séparément la solution aqueuse de gomme carboxyméthylée et la solution contenant le sel (ou les sels) de chrome. Si l'on veut retarder la prise en gel, on utilise un séquestrant tel que l'hexamétaphosphate de sodium dans la solution de gomme ou dans la solution de sel. On mélange ensuite les deux solutions dans les proportions voulues.

Le gel prend plus ou moins rapidement suivant la nature de sel de chrome, la quantité de séquestrant et aussi suivant les proportions de sel métallique et de gomme. Il est donc possible de maîtriser le temps de prise en gel.

Pour la fabrication d'un gel, on peut par les procédés connus, par exemple celui décrit dans le brevet US 2 599 771; on peut aussi opérer avec la technique et l'appareillage suivants:

- dans un conteneur A muni d'un agitateur, on introduit de l'eau, le galactomannane carboxyméthylé, un agent de conservation tel que l'acide benzoïque ou les benzoates et on agite jusqu'à l'obtention d'une solution limpide,
- dans un conteneur B muni d'un agitateur, on introduit de l'eau et le (ou les) sel de chrome, le tout sous bonne agitation.

Chaque conteneur est relié à une pompe doseuse. Ces deux pompes sont synchronisées et réglées de façon qu'à chaque cycle la quantité de solution A et la quantité de solution B sont telles que l'on a un rapport pondéral chrome sur gomme carboxyméthylée compris entre 0,005 et 0,1.

Les deux fractions de solutions passent ensuite dans un mélangeur statique (ou dans une cuve de mélange munie d'un agitateur). Les matériaux ainsi mélangés sont versés dans des récipients individuels pendant qu'ils sont encore fluides et on les laisse réagir en place pour former un gel solide ne s'affaissant pas.

Ce procédé permet de former des gels de traitement de l'air ayant de bonnes caractéristiques de cohésion, d'élasticité, de comparibilité avec les constituants habituels de ce type de gel (colorants, parfums . . .) et exempts de synérèse.

En particulier, il a été constaté que l'on pouvait produire un gel stable pour traitement de l'air en utilisant une composition contenant de 0,5% à 10% en poids de galactomannane carboxyméthylé, un parfum, un colorant, une source de cation chrome en quantité telle que le rapport quantité de métal trivalent sur quantité de gomme carboxyméthylée soit compris entre 0,005 et 0,1 et de l'eau. Le gel obtenu est tel que si on l'expose à l'atmosphère et qu'on laisse l'eau s'évaporer il libère le parfum pendant une période de temps étendue.

L'invention sera mieux comprise par les exemples, ci-dessous, de formules donnant des gels satisfaisants.

Exemple 1

On prépare un gel solide à partir de la composition suivante:

| | | |
|---|---|---|
| Solution A | — eau | 210 g |
| | — gomme de guar carboxyméthylée type Na | 9,45 g |
| | — agent de conservation | 0,20 g |
| Solution B | — eau | 80 g |
| | — chlorure de chrome $CrCl_3$ 6 $H_2$ | 1,02 g |

La solution B est ajoutée à la solution A sous bonne agitation et l'on coule la solution dans les récipients où l'on veut obtenir les gels. La gélification intervient après 2 à 3 minutes. Après repos, on obtient un gel rigide et exempt de synérèse.

Exemple 2

On prépare un gel solide à partir de la composition suivante:

| | | |
|---|---|---|
| Solution A | — eau | 210 g |
| | — gomme tara carboxyméthylée type K | 9,75 g |
| | — agent de conservation | 0,20 g |
| Solution B | — eau | 80 g |
| | — chlorure de chrome $CrCl_3$ 6 $H_2O$ | 1,02 g |

La solution B est ajoutée à la solution A sous bonne agitation et le mélange est coulé dans des récipients individuels. Après repos, on obtient un gel rigide et exempt de synérèse.

Exemple 3

On prépare un gel solide à partir de la composition suivante:

| | | |
|---|---|---|
| Solution A | — eau | 210 g |
| | — gomme d'Espina corona carboxyméthylée type Na | 9,50 g |
| | — agent de conservation | 0,20 g |
| Solution B | — eau | 80 g |
| | — chlorure de chrome | 0,51 g |
| | — sulfate de chrome | 0,51 g |

On ajoute la solution B à la solution A sous forte agitation. La gélification apparaît après 15 minutes. Le gel obtenu, après repos, est ferme et sans synérèse.

La solution B est ajoutée à la solution A sous bonne agitation et le mélange est coulé dans des récipients individuels. Après repos, on obtient un gel rigide et exempt de synérèse.

Exemple 4

On suit le mode opératoire de l'exemple 1 sauf que l'on remplace le chlorure de chrome par du sulfate de chrome. La gélification intervient après 20 minutes. Après repos, on obtient un gel rigide exempt de synérèse.

Exemple 5

On répète l'exemple 3 en ajoutant 0,05% en poids d'hexamétaphosphate de sodium dans la solution A. La gélification intervient après 1 heure environ. Le gel ne présente aucune synérèse.

Exemple 6

Influence de la concentration

On a mesuré la force de gel pour diverses concentrations d'une gomme de guar carboxyméthylée (CM guar) d'un degré de substitution donné utilisé avec un rapport $Cr^{3+}$/CM guar de 0,023/1.

La force de gel a été mesurée à l'appareil Bloomgelometer avec une course de piston de 8 mm.

Les résultats sont donnés dans le tableau 1 ci-dessous:

Tableau 1

| Concentration | Quantités mises en jeu | | Rapport | Force de gel |
|---|---|---|---|---|
| | CM guar | $CrCl_3$ | $Cr^{3+}$/CM guar | |
| 1,5% | 4,59 g | 0,510 g | 0,023/1 | 75 |
| 2% | 6,12 g | 0,680 g | 0,023/1 | 105 |
| 2,5% | 7,65 g | 0,850 g | 0,023/1 | 155 |
| 3% | 9,18 g | 1,020 g | 0,023/1 | 230 |

Exemple 7

Influence du degré de substitution (DS) de la gomme de guar carboxyméthylée (CM guar)

La force de gel a été mesurée avec le même appareil que dans l'exemple 6 à la même concentration de 3% en CM guar réticulé par le chlorure de chrome selon un rapport $Cr^{3+}$/CM guar de 0,023/1.

Les résultats sont donnés dans le tableau 2 ci-dessous:

Tableau 2

| DS | Concentration | Rapport $Cr^{3+}$/CM guar | Force de gel |
|---|---|---|---|
| 0,25 | 3% | 0,023/1 | 80 |
| 0,50 | 3% | 0,023/1 | 230 |
| 1 | 3% | 0,023/1 | 360 légère synérèse |
| 2 | 3% | 0,023/1 | non mesuré synérèse |

On obtient une force de gel croissante lorsque le degré de substitution augmente; néanmoins avec un degré de substitution élevé il y a formation de synérèse et le gel devient plus cassant.

### Exemple 8
### Influence du degré de polymérisation de la gomme de guar carboxyméthylée (CM guar)

Plus le degré de polymérisation est élevé, plus la viscosité est élevée; on apprécie donc le degré de polymérisation par la mesure de la viscosité, au viscosimètre Brookfield RVT 20 RPM, d'une solution de 1% de CM guar d'un degré de substitution (DS) de 1 et réticulée par le chlorure de chrome selon un rapport $Cr^{3+}$/CM guar de 0,023/1.

Le tableau 3 ci-dessous donne les résultats de deux mesures, la force de gel étant toujours mesurée comme dans l'exemple 6.

Tableau 3

| DS | Viscosité centipoises | Concentration | Rapport $Cr^{3+}$/CM guar | Force de gel |
|---|---|---|---|---|
| 1 | 400 | 2% | 0,023/1 | 280 légère synérèse |
| 1 | 40 | 2% | 0,023/1 | 135 |

### Exemple 9
### Influence de la quantité de chlorure de chrome

On a opéré une gomme de guar carboxyméthylée d'un degré de substitution DS de 0,25 à une concentration de 3% et mesuré la force de gel comme dans l'exemple 7; de plus, on a apprécié la synérèse du gel au bout de 96 heures (synérèse 96 h).

Les résultats sont donnés dans le tableau 4 ci-dessous:

Tableau 4

| DS | Concentration | Rapport $Cr^{3+}$/CM guar | Force de gel | Synérèse 96 h |
|---|---|---|---|---|
| 0,25 | 3% | 0,023/1 | 65 | aucune |
| 0,25 | 3% | 0,025/1 | 70 | aucune |
| 0,25 | 3% | 0,027/1 | 85 | aucune |
| 0,25 | 3% | 0,032/1 | 115 | aucune |
| 0,25 | 3% | 0,034/1 | 135 | aucune |

Exemple 10

On a refait l'essai précédent mais avec un degré de substitution (DS) de 1 et à une concentration de 2 pour cent.

Les résultats sont donnés dans le tableau 5 ci-dessous:

Tableau 5

| DS | Concentration | Rapport $Cr^{3+}$/CM guar | Force de gel | Synérèse 96 h |
|---|---|---|---|---|
| 1 | 2% | 0,010/1 | 25 | aucune |
| 1 | 2% | 0,017/1 | 80 | aucune |
| 1 | 2% | 0,023/1 | 135 | aucune |
| 1 | 2% | 0,034/1 | 435 | importante |
| 1 | 2% | 0,050/1 | non mesurée | précipitation |

D'après les exemples 9 et 10, on remarque qu'avec un faible DS de la gomme de guar carboxyméthylée, on peut utiliser des doses de sel de métal trivalent élevées, sans pour cela avoir de synérèse.

Au contraire, une gomme de guar carboxyméthylée d'un DS élevé est plus sensible à l'influence du cation trivalent dont des doses importantes provoquent l'apparition de synérèse.

Exemple 11

On prépare un gel solide de traitement de l'air à partir de la composition suivante:

| | | |
|---|---|---|
| Solution A | — eau | 210 g |
| | — gomme de guar carboxyméthylée type Na | 9,45 g |
| | — agent de conservation | 0,20 g |
| Solution B | — eau | 80 g |
| | — colorant | 0,25 g |
| | — parfum* | 3,5 g |
| | — chlorure de chrome $CrCl_3$ 6 $H_2O$ | 1,02 g |

* Le parfum utilisé doit être compatible avec l'eau (sous forme émulsionnée).

Cette composition forme un gel ferme et souple qui, par repos, ne forme pas de synérèse et libère le parfum sur une période de temps convenable.

**Revendications**

1. Utilisation comme gels de supports de produits de traitement de l'air, de compositions dans lesquelles l'agent gélifiant principal est un galactomannane carboxyméthylé réticulé par des cations chrome.

2. Utilisation de gels de support selon la revendication 1, caractérisée par ceci que le galactomannane carboxyméthylé est obtenu à partir:

— de la gomme de guar (Cyamopsis tetragonolobus),
— de la gomme de caroube (Ceratonia siliqua),
— de la gomme de tara (Caesalpinia spinosa),
— de la gomme d'Espina corona (Gleditsia amorphoïdes)

utilisées seules ou en mélange.
3. Utilisation de gels de support selon les revendications 1 ou 2, caractérisée par ceci que ledit galactomannane carboxyméthylé est sous forme sodique ou potassique.
4. Utilisation de gels de support selon les revendications 1 à 3, caractérisée par ceci que le galactomannane carboxyméthylé est utilisé à une concentration comprise entre 0,5% et 10% en poids.
5. Utilisation de gels de support selon les revendications 1 à 4, caractérisée par ceci que la source de cations chrome est choisie parmi le groupe des sels suivants:

— sulfate de chrome, sulfate basique de chrome, chlorure de chrome, acétate de chrome, alun de chrome.

6. Utilisation de gels de support selon les revendications 1 à 5, caractérisée par ceci que les sels de chrome sont utilisés seuls ou en mélange.
7. Utilisation de gels de support selon les revendications 1 à 6, caractérisée par ceci que le rapport pondéral entre le cation chrome et la gomme carboxyméthylée est compris entre 0,005 et 0,1.
8. Utilisation de gels de support selon les revendications 1 à 7, caractérisée par le fait qu'on utilise de 0 à 1% d'un agent de conservation tel que l'acide benzoïque et les benzoates.
9. Utilisation d'agent gélifiant selon les revendications 1 à 8, dans les gels de support de produits de traitement de l'air libérant des composés tels que:

— désinfectants,
— bactéricides,
— insecticides,
— parfums ou huiles essentielles,
— désodorisants.

**Patentansprüche**

1. Verwendung von Zubereitungen, in denen ein durch Chromkationen vernetztes carboxymethyliertes Galactomannan der Hauptgelbildner ist, als Trägergele für Produkte zur Behandlung von Luft.
2. Verwendung nach Anspruch 1, dadurch gekennzeichnet, daß das carboxymethylierte Galactomannan hergestellt wurde aus:

— Guar (Cyamopsis tetragonolobus-)Gummi,
— Johannisbrot (Ceratonia siliqua-)Gummi,
— Tara (Caesalpinia spinosa-)Gummi,
— Espina corona (Gleditsia amorphoides-)Gummi,

die einzelnen oder in Form eines Gemisches verwendet wurden.
3. Verwendung nach Anspruch 1 oder 2, dadurch gekennzeichnet daß das carboxymethylierte Galactomannan in Form des Natrium- oder Kaliumsalzes vorliegt.
4. Verwendung nach den Ansprüchen 1 bis 3, dadurch gekennzeichnet, daß das carboxymethylierte Galactomannan in einer Konzentration zwischen 0,5 und 10 Gew.-% verwendet wird.
5. Verwendung nach den Ansprüchen 1 bis 4, dadurch gekennzeichnet, daß die Quelle für Chromkationen ausgewählt wird aus der Gruppe der folgenden Salze:

— Chromsulfat, basisches Chromsulfat, Chromchlorid, Chromacetat, Chromalaun.

6. Verwendung nach den Ansprüchen 1 bis 5 dadurch gekennzeichnet, daß die Chromsalze einzeln oder in Form eines Gemisches verwendet werden.
7. Verwendung nach den Ansprüchen 1 bis 6, dadurch gekennzeichnet, daß das Gewichtsverhältnis zwischen dem Chromkation und dem carboxymethylierten Gummi zwischen 0,005 und 0,1 liegt.
8. Verwendung nach den Ansprüchen 1 bis 7, dadurch gekennzeichnet, daß 0 bis 1% eines Konservierungsmittels, wie Benzoesäure und die Benzoate, verwendet wird.
9. Verwendung eines Gelbildners nach den Ansprüchen 1 bis 8 in Trägergelen für Produkte zur

Behandlung von Luft, die Verbindungen freisetzen, wie z. B. in

- Desinfektionsmitteln,
- Bakteriziden,
- Insektiziden,
- Parfüms oder essentiellen Ölen,
- Deodorantien.

## Claims

1. Use as support gels of products of treating with air compositions in which the principal gelling agent is a carboxymethylated galactomannan cross-linked by means of cations of chromium.

2. Use of support gels according to claim 1, characterised in that the carboxymethylated galactomannan has been obtained from:

- guar gum (Cyamopsis tetragonolobus),
- carouba gum (Ceratonia siliqua),
- tara gum (Caesalpinia spinosa),
- Espina corona gum (Gleditsia amorphoides)

used alone or in admixture.

3. Use of support gels according to claim 1 or 2, characterised in that the said carboxymethylated galactomannan is in the sodium potassium form.

4. Use of support gels according to claims 1 to 3, characterised in that the carboxymethylated galactomannan is used in a concentration between 0.5% and 10% by weight.

5. Use of support gels according to claim 1 to 4, characterised in that the source of chromium cations is selected from the following salts:

- chromium sulphate, basic chromium sulphate, chromium chloride, chromium acetate, chrome alum.

6. Use of support gels according to claims 1 to 5, characterised in that the chromium salts are used alone or in admixture.

7. Use of support gels according to claim 1 to 6, characterised in that the weight relationship between the chromium cation and the carboxymethylated gum amounts to between 0.005 and 0.1.

8. Use of support gels according to claim 1 to 7, characterised in that there is utilized therein from 0 to 1% of a preservative such as benzoic acid and benzoates.

9. Use of gelling agents according to claim 1 to 8, in the support gels of products for the treatment of air liberating compounds such as:

- disinfectants,
- bactericides,
- insecticides,
- perfumes or essential oils,
- deoderants.